Europäisches Patentamt

(19) European Patent Office

Office européen des brevets

(11) Numéro de publication: **0 066 505**
**B1**

(12) **FASCICULE DE BREVET EUROPEEN**

(45) Date de publication du fascicule du brevet:
13.02.85

(51) Int. Cl.⁴: **A 61 K 9/26**

(21) Numéro de dépôt: **82400931.0**

(22) Date de dépôt: **19.05.82**

(54) **Préparation pharmaceutique de "naftidrofuryl" à libération contrôlée.**

(30) Priorité: **20.05.81 FR 8110060**

(43) Date de publication de la demande:
**08.12.82 Bulletin 82/49**

(45) Mention de la délivrance du brevet:
**13.02.85 Bulletin 85/7**

(84) Etats contractants désignés:
**AT BE CH DE FR GB IT LI LU NL SE**

(56) Documents cités:
**FR - A - 1 416 304**
**FR - A - 2 377 196**
**US - A - 3 062 720**

**CHEMICAL ABSTRACTS, vol. 94, 1981, page 371, no. 71384e, Columbus Ohio (USA); J. SHANI et al.: "Sustained release of radioprotective agents in vitro".**
**CHEMICAL ABSTRACTS, vol. 91, 1979, page 322, no. 96579y, Columbus Ohio (USA); C. BOYMOND et al.: "Study of the effect of glycerol palmitostearate on the release and solution kinetics of ephedrine hydrochloride"**

(73) Titulaire: **LIPHA, LYONNAISE INDUSTRIELLE PHARMACEUTIQUE, 115, avenue Lacassagne B.P. No 106 Lyon R.P., F-69212 Lyon Cedex 1 (FR)**

(72) Inventeur: **Lechevin, Jean-Claude, 56, rue de la Charité, F-69002 Lyon (FR)**
Inventeur: **Bellevegue, Monique, 127, rue de la pagère, F-69500 Bron (FR)**

(74) Mandataire: **Bouton Neuvy, Liliane et al, L'Air liquide, Société Anonyme pour L'Etude et L'Exploitation des Procédés Georges Claude 75, Quai d'Orsay, F-75321 Paris Cedex 07 (FR)**

## Description

La présente invention concerne une préparation pharmaceutique de »Naftidrofuryl« à libération contrôlée.

Le médicament contenant comme principe actif le (naphtyl-1)-3 tétrahydrofurfuryl-2 proprionate de diéthylamino-2 éthyle, communément désigné »Naftidrofuryl« est connu pour son action dans le traitement des troubles circulatoires périphéripues et cérébraux.

Sous forme de base libre et de sel utilisable en thérapeutique il fait l'objet des certificat d'addition 83 555, brevet d'invention 1 363 948 et brevet spécial de médicament 3843 M au nom de la demanderesse.

L'administration par voie orale se fait usuellement sous forme de capsule dure de gélatine, forme pharmaceutique à action immédiate, ne permettant pas le maintien d'un taux sanguin efficace suffisamment longtemps pour réduire le nombre de prises à deux par jour.

Il est trés important de prolonger l'activité thérapeutique en assurant une couverture égale et continue notamment de la circulation cérébrale tout au long du nycthémère.

La demande de brevet d'invention 2 377 196 décrit une composition médicamenteuse de Naftidrofuryl à activité thérapeutique prolongée, présentée en comprimés enrobés qui pallie les inconvénients d'une arrivée massive de principe actif suivie d'une chute de l'effet thérapeutique par disparition du principe actif dans le fond circulant.

La demande de brevet d'invention 2 453 639 a pour objet une présentation galénique de Naftidrofuryl à libération programmée du principe actif, comprenant des microgranules à libération immédiate formées chacune d'un grain support, auquel est fixé du Naftidrofuryl et des microgranules à libération programmée formées chacune d'un grain support, auquel est fixé du Naftidrofuryl enrobé d'un enrobage en un polymère de l'acide méthacrylique ou en polyméthacrylate.

Certaines formules de comprimés retard ont déjà été proposées, chacune étant choisie en fonction du principe actif. Ainsi, le brevet américain 3 062 720, pour des substances solubles dans l'eau, telles le sufate de d-amphétamine, le pentylènetétrazole, l'acide nicotinique, le sulfate d'hyoscymine, le sulfate d'atropine, l'hydrobromure d'hyoscine et le pentobarbital de sodium, et pour un retard d'au plus huit heures, suggère d'associer le médicament à un produit gras insoluble dans l'eau, tel les stéarates de calcium et de magnésium et le glycérylmonostéarate, à un excipient, un liant notamment les alkylcelluloses sous forme monomère, et un lubrifiant. L'étude de l'Université hébraïque de Jérusalem (C. A Vol 94, 1981, p. 371) concerne l'effet retard de médicament radioprotecteur du type acide aminé soufré dans des matrices d'acide stéarique et d'éthylcellulose. Et le laboratoire de pharmacie galénique de Strasbourg (C. A vol 91, 1971, p. 322) a étudié l'effet retard du palmitostéarate de glycérol sur l'éphédrine connue notamment pour son action cardiovasculaire et son action sur la respiration.

Il a été trouvé une préparation pharmaceutiquee spécialement adaptée au Naftidrofuryl et ses sels, permettant d'assurer une couverture thérapeutique entre deux prises séparées de 12 heures correspondant au maintien d'un taux sanguin significatif stable élevé et d'augmenter de façon considérable la biodisponibilité du médicament.

La diminution de la vitesse de dissolution du Naftidrofuryl et de ses sels organiques et minéraux pharmaceutiquement acceptables est obtenue par inclusion du principe actif dans une matrice hydrophobe, à partir de laquelle il diffuse lentement, assurant ainsi une absorption régulière et prolongée.

La matrice hydrophobe est constituée par le mélange d'une substance lipidique et d'un polymère sellulosique insoluble dans l'eau.

La substance lipidique est le palmitostéarate de sorbitol et de glycérol. La quantité de substance lipidique utilisée représente 15 à 30% du poids du Naftidrofuryl.

Le polymère cellulosique est un alkylcellulose, en particulier l'éthylcellulose, la quantité de polymère cellulosique insoluble utilisée représente 5 à 15% du poids du Naftidrofuryl.

La préparation pharmaceutique peut être conditionnée sous forme de comprimé et la formule du comprimé peut associer au principe actif et aux excipients destinés à limiter la vitesse de diffusion, d'autres excipients classiques : diluants, désintégrants, et labrifiants. Le choix du diluant permet également de régler selon l'hydrophilie la vitesse de diffusion.

Le comprimé peut être fabriqué classiquement par le procédé de granulation humide.

On peut avantageusement procéder à l'application d'un enrobage gastrorésistant et ledit enrobage est réalisé au moyen de substances utilisées à cet effet, tel le phtalate d'hydroxypropylméthyl cellulose.

La mesure in vitro de la vitesse de dissolution du principe actif est effectuée à 37° C dans un liquide d'épreuve de pH 6,8. La composition du comprimé selon l'invention est telle que la quantité de principe actif dissoute après 15 heures soit comprise entre 80% et 100%.

La comparaison in vivo de la forme pharmaceutique à action immédiate ( capsule dure de gélatine) et du comprimé selon l'invention, est faite sur l'homme par mesure du taux sanguin et calcul de l'aire sous lacourbe pour la détermination de la biodisponibilité. Le Naftidrofuryl plasmatique est dosé par spectrométrie d'absorption U. V. à 222 nm après extraction et séparation par chromatographie liquide haute performance.

Il est donné ci-aprés, à titre non limitatif, un exemple qui illustre l'invention:

Exemple

On mélange 20 kg de Naftidrofuryl avec 6 kg de palmitostéarate de sorbitol et de glycérol, 3 kg d'éthyl-cellulose 20 cps et 27,7 kg de lactose. Le mélange obtenu est mouillé, granulé, puis séché. On ajoute au granulé calibré 0,6 kg d'acide alginique, 0,2 kg de dioxyde de silicium, 0,7 kg de talc et 0,8 kg de stéarate de magnésium. Le mélange obtenu est pressé pour obtenir des comprimés, d'un poids unitaire de 590 mg. Chaque comprimé contient 200 mg de Naftidrofuryl. Les comprimés sont enrobés en turbine avec une solution de phtalate d'hydroxypropylméthylcellulose de manière à obtenir la gastrorésistance recherchée.

La courbe de dissolution in vitro à pH 6,8 donne les résultats suivants:

| Temps d'étude | % Naftidrofuryl dissous |
|---|---|
| 1 h | 15% |
| 3 h | 41% |
| 7 h | 72,5% |
| 15 h | 88,5% |

L'étude in vivo est réalisée sur 12 sujets de sexe masculin chez lesquels on compare en administration répétée la capsule dure de gélatine et le comprimé selon l'invention. L'étude est faite en administration répétée pendant 7 jours de manière à se placer dans des conditions d'équilibre comparables à celles obtenues au cours du traitement des malades. Chacun des sujets reçoit successivement, dans un ordre aléatoire, chacune des deux formes pharmaceutiques. Un intervalle d'une semaine, sans administration, est laissé entre les deux séquences successives. Pour chaque séquence, du premier au sixième jour, chacun des sujets absorbe par voie orale, matin et soir, 200 mg de Naftidrofuryl. Le septième jour, un premier prélèvement de sang veineux est fait (temps 0) avant la prise du médicament du matin et il n'y aura pas de prise de médicament le soir. Les autres prélèvements veineux sont effectués 1 h, 2 h, 3 h, 6 h, 8 h, 10 h, 12 h, 15 h et 24 h après la dernière prise du médicament. Les résultats obtenus sont rassemblés dans le tableau suivant:

| Temps d'étude Zéme jour | Naftidrofuryl plasmatique en ng/ml aprés 200 mg de Naftidrofuryl | |
|---|---|---|
| | capsule dure de gélatine | comprimé selon l'invention |
| 0 | 0 | 257,7 |
| 1 h | 970,5 ± 116,4 | 234 ± 22,5 |
| 2 h | 469,6 ± 73,5 | 235,6 ± 25,1 |
| 3 h | 123,2 ± 14,8 | 330 ± 41,6 |
| 6 h | 35,4 ± 7,4 | 361,2 ± 32,3 |
| 8 h | 6,8 ± 1,8 | 400,7 ± 33,7 |
| 10 h | 0 | 369,8 ± 28,3 |
| 12 h | 0 | 284,8 ± 29,7 |
| 15 h | 0 | 141,3 ± 20,9 |
| 24 h | 0 | 26,3 ± 7,2 |
| Aire sous la courbe en ng/ml$^{-1}$/hr | 1878,5 ± 41,6 | 5555,8 ± 380,11 |

L'examen de ce tableau montre que

1. La capsule dure de gélatine donne une concentration sanguine avec un pic suivi d'une rapide diminution; il n'y a ainsi qu'une relativement courte période de temps pendant laquelle on observe un taux sanguin significatif. Le comprimé selon l'invention donne, au contraire, un taux sanguin durable et homogène dans le temps.

2. Avec une prise matin et soir, le taux sanguin au temps 0, donc antérieurement à la prise matinale, est voisin de 250 ng/ml pour le comprimé selon l'invention, alors qu'il est nul avec la capsule dure de gélatine. La capsule dure de gélatine donne des pics sanguins successifs séparés par de longues périodes où le sang ne contient plus le médicament. Le comprimé selon l'invention assure, avec une prise matin et soir, une imprégnation permanente de l'organisme au cours des 12 heures séparant les deux prises, le taux sanguin ne descendant pas au-dessous de 234 ng/ml et ne montant pas audessus de 400 ng/ml.

En terme pharmacocinétique, les »aires sous la courbe« expriment, pour chacune des deux formes, la biodisponibilité. Le rapport des aires sous la courbe a la valeur suivante:

$$\frac{\text{Aire sous la courbe comprimé selon l'invention}}{\text{Aire sous la courbe capsule dure de gélatine}} = \frac{5555,8}{1878,5} = 2,958$$

Cela signifie que le comprimé selon l'invention possède une biodisponibilité 3 fois supérieure à celle de la capsule dure de gélatine. Cette augmentation de biodisponibilité est exceptionnelle et bien supérieure à celle de la forme galénique de Naftidrofuryl à action instantanée ratard, décrite dans la demande de brevet 2 453 639. Le rapport des aires sous la courbe y a la valeur suivante:

$$\frac{4,268}{2,849} = 1,498,$$

ce qui signifie une augmentation de biodisponibilité de 1,5 fois.

La nouvelle préparation pharmaceutique à libération contrôlée permettant une bonne couverture thérapeutique est utilisée dans le traitement des troubles circulatoires périphériques et cérébraux.

## Revendications

1. Préparation pharmaceutique de Naftidrofuryl à libération contrôlée, caractérisée en ce que le Naftidrofuryl, ses sels organiques ou minéraux acceptables en thérapeutique sont inclus dans une matrice hydrophobe, constituée par le palmitostéarate de sorbitol et de glycérol représentant 15 à 30% du poids de Naftidrofuryl et d'un polymère insoluble dans l'eau de la classe des alkylcelluloses, représentant 5 à 15% du poids de Naftidrofuryl.

2. Préparation pharmaceutique de Naftidrofuryl selon la revendication 1, caractérisée en ce que le polymère cellulosique est l'éthylcellulose.

3. Préparation pharmaceutique de Naftidrofuryl selon la revendication 1, caractérisée en ce que la dite préparation est conditionnée sous forme gastro-résistante, en particulier de comprimé gastro-résistant.

## Patentansprüche

1. Pharmazeutisches Naftidrofurylpräparat mit verzögerter Freisetzung des Wirkstoffes, dadurch gekennzeichnet, daß das Naftidrofuryl oder seine therapeutisch verträglichen organischen oder mineralischen Salze in einer hydrophoben Matrix eingeschlossen sind, die aus Sorbit- und Glycerinpalmitostearat in einer Menge von 15 bis 30 Gew.-% des Naftidrofuryls und einem in Wasser unlöslichen Polymer der Klasse der Alkylcellulosen in einer Menge von 5 bis 15 Gew.-% des Naftidrofuryls besteht.

2. Pharmazeutisches Naftidrofurylpräparat nach Anspruch 1, dadurch gekennzeichnet, daß das Cellulosepolymer Äthylcellulose ist.

3. Pharmazeutisches Naftidrofurylpräparat nach Anspruch 1, dadurch gekennzeichnet, daß dieses Präparat in magensaftbeständiger Form, besonders als magensaftbeständige Tablette, zubereitet ist.

**Claims**

1. Pharmaceutical sustained-release preparation of Naftidrofuryl, characterized in that the Naftidrofuryl or its therapeutically acceptable organic or mineral salts are included in a hydrophobic matrix consisting of sorbitol and glycerol palmito stearate representing 15 to 30 percent by weight of Naftidrofuryl and of a polymer insoluble in water of the class of the alkyl celluloses representing 5 to 15 percent by weight of Naftidrofuryl.

2. Pharmaceutical preparation of Naftidrofuryl according to claim 1, characterized in that the celulosic polymer is ethyl cellulose.

3. Pharmaceutical preparation of Naftidrofuryl according to claim 1, characterized in that the said preparation is conditioned in gastro-resistant form, especially as gastro-resistant tablet.